# EUROPEAN PATENT APPLICATION

(11) **EP 3 222 211 A1**
(43) Date of publication of application: **27.09.2017**
(21) Application number: 16173469.4
(22) Date of filing: 08.06.2016
(51) Int. Cl.: A61B 5/021

(54) **BLOOD PRESSURE MEASUREMENT DEVICE AND METHOD OF BLOOD PRESSURE MEASUREMENT**

(30) Priority: 23.03.2016 TW 105109046
(71) Applicant: Maisense Inc., Hsinchu County 30273 (TW)
(72) Inventor: LEE, Yung-Pin, 23581 Zhonghe Dist., New Taipei City (TW)
(74) Representative: Zimmermann, Tankred Klaus

(57) **Abstract**

A blood pressure measurement device comprises a case, a first electrode and a stress sensor. The case includes a first surface and a second surface. The first electrode is disposed on the first surface, and the stress sensor is disposed on the second surface. The blood pressure measurement device is operated on a part of a body of a user. The part of the body has a blood vessel inside. Upon measurement of the blood pressure, the first electrode, the stress sensor and the blood vessel at least partially overlap in one projection orientation. Therefore, the stress sensor is able to be pressed on the part of the body when the user touches the first electrode. A method of blood pressure measurement is also disclosed.

## Description

### BACKGROUND OF THE INVENTION

### Field of Invention

The invention relates to a blood pressure measurement device and a method of blood pressure measurement.

### Related Art

The blood pressure is one of important indicators for the observation of the individual's health condition. The abnormal value of the blood pressure, either the too-high or too-low value, implies that a problem of the individual's physiological condition, especially the cardiovascular disease that is difficult to be observed from the outlook of the individual, arises. Because the elder's body has the aging function, there is a high risk of suffering from the cardiovascular disease, and it is a very important work to regularly measure the blood pressure of the elder so that the disease can be detected and treated early. However, the modern human beings have the high life pressure, and the irregular three meals. Thus, the group of persons whose blood pressures need to be regularly monitored has the decreasing age.

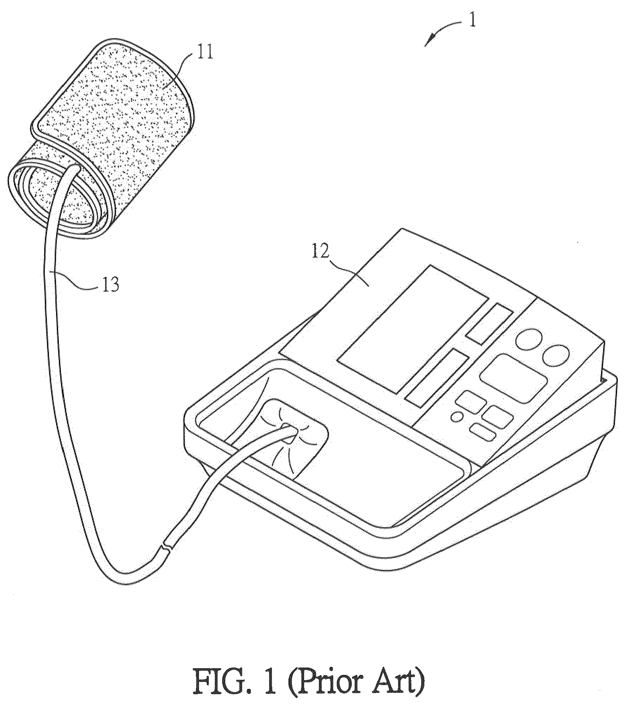

FIG. 1 is a schematic view showing an exterior of a conventional sphygmomanometer. Referring to FIG. 1, a sphygmomanometer 1 comprises a cuff 11, a host 12 and a rubber tube 13. Upon measurement of the blood pressure, the host 12 controls the inflating pressurization and the deflating depressurization on the cuff 11 through the rubber tube 13, and substitutes the pressure fluctuation value, which represents vibration amplitude of the blood vessel wall and is sensed by the pressure sensor contained in the cuff 11, into the oscillometry to calculate the blood pressure in this process.

However, the conventional sphygmomanometer 1 tends to make the subject feel uncomfortable in the pressurization and depressurization processes, and needs the too-long measurement time, so that the subject's measurement desire is low, and it is disadvantageous to the long-term use. In addition, regarding the portable property, the sizes of the host 12 and the cuff 11 are too large, and are disadvantageous to the monitoring of the blood pressure at any time.

Recently, some watch-type blood pressure measurement devices are available in the market. Although this new product has overcome the problem of the portable property of the conventional sphygmomanometer 1, the product cannot be properly fixed at the measurement location and thus cannot precisely measure the pulse signal of the blood vessel. Thus, the blood pressure measurement value is not precise, or even the measurement failure may occur.

Therefore, it is a great need to provide a blood pressure measurement device, which can be easily carried, has the rapid measurement process without causing the uncomfortable problem upon pressurization and depressurization, and can be easily fixed at the measurement location to enhance the accuracy and success rate of measuring the blood pressure.

### SUMMARY OF THE INVENTION

In view of the above-identified subject, an object of the invention is to provide a blood pressure measurement device and a method of blood pressure measurement. The host and the cuff, which are necessary for the pressurization and depressurization, are omitted according to the design of the device, so that the size of the device can be reduced and can be easily carried by the user to enhance the desire of the measurement of the blood pressure and further to advantageously achieve the monitoring of the blood pressure at any time. More importantly, the invention can make the user easily fix the device at the measurement location while obtaining the measurement signal and thus provide an instinctive operation. Thus, the blood pressure can be precisely measured, the requirement (e.g., the adjustment made according to the algorithm after the blood pressure is measured) on subsequently using the software to perform the calibration is reduced. In addition, the problem (e.g., using the additional mechanism to strengthen the fixing of the device at the measurement location) that the user needs to learn specially can be prevented.

To achieve the above objective, the present invention discloses a blood pressure measurement device, which includes a case, a first electrode and a stress sensor. The case includes a first surface and a second surface. The first electrode is disposed on the first surface, and the stress sensor is disposed on the second surface. The blood pressure measurement device is operated on a part of a body of a user, which has a blood vessel inside. Upon measurement of a blood pressure, the first electrode, the stress sensor and the blood vessel at least partially overlap in one projection orientation, so that when the user touches the first electrode, the stress sensor is able to be pressed on the part of the body.

To achieve the above objective, the present invention also discloses a method of blood pressure measurement, which includes the following steps of: using a blood pressure measurement device to contact a part of a body of a user, wherein the blood pressure measurement device includes a case, a first electrode and a stress sensor, the case includes a first surface and a second surface, the first electrode is disposed on the first surface, the stress sensor is disposed on the second surface, and the part of the body has a blood vessel inside; making the first electrode, the stress sensor and the blood vessel at least partially overlap in one projection orientation; and making the user touch the first electrode so that the stress sensor is able to be pressed on the part of the body.

In one embodiment, when the user touches the first electrode, the stress sensor encounters a downward pressing force generated by a gravity force exerted to the user or an exertion force exerted by the user and is able to be pressed on the part of the body.

In one embodiment, the blood pressure measurement device further includes a second electrode, which is disposed on the second surface and is electrically connected to the first electrode. Upon measurement of the blood pressure, the second electrode contacts the part of the body.

In one embodiment, when the user touches the first electrode, the second electrode is able to be pressed on the part of the body.

In one embodiment, the blood pressure measurement device further includes a calibration electrode, one or multiple processing units and a storage unit. The calibration electrode is disposed on the case. The one or multiple processing units accommodated within the case. The storage unit is accommodated within the case and is signal connected to the one or multiple processing units. The storage unit includes one or multiple program instructions. When the one or multiple program instructions are executed by the one or multiple processing units, the one or multiple processing units execute the steps of: obtaining a first calibration value, wherein the first calibration value is calculated according to a first measured result of the calibration electrode and of the first electrode; obtaining a second calibration value, wherein the second calibration value is calculated according to a second measured result of the calibration electrode and the first electrode; and calibrating measured values obtained by the first electrode and the second electrode according to the first calibration value and the second calibration value.

In one embodiment, the user uses a finger to touch the first electrode, and the first electrode has a concave portion corresponding to a shape of the finger.

As mentioned above, the invention provides a blood pressure measurement device and a method of blood pressure measurement, in which the blood pressure is calculated according to the two electrodes and the sensing signal of the stress sensor without the need of the host and the cuff for pressurization and depressurization. Thus, the size can be advantageously decreased so that the blood pressure measurement device can be easily carried, and the user's desire of monitoring the blood pressure can be enhanced. More importantly, the mechanism is designed such that the first electrode, the stress sensor and the blood vessel in the to-be-measured part of the body at least partially overlap in one projection orientation. Thus, when the user is operating the device, the user only needs to touch the first electrode at his/her convenience to make the stress sensor press upon the to-be-measured part of the body. Thus, the measurement precision can be enhanced, and the requirement of the subsequent calibration using the software is decreased. More particularly, an instinctive operation can be provided to achieve the good measurement effect without the particular learning.

### BRIEF DESCRIPTION OF THE DRAWINGS

The invention will become more fully understood from the detailed description and accompanying drawings, which are given for illustration only, and thus are not limitative of the present invention, and wherein:
FIG. 1 is a schematic view showing a conventional inflatable sphygmomanometer;
FIG. 2A is a schematic view showing an exterior of a blood pressure measurement device according to a first embodiment of the invention;
FIG. 2B is a schematic system block diagram showing the blood pressure measurement device according to the first embodiment of the invention;
FIGS. 3A to 3C are schematic views showing operations of the blood pressure measurement device according to the first embodiment of the invention;
FIGS. 4A and 4B are schematic views showing elements of the blood pressure measurement device according to the first embodiment of the invention;
FIG. 4C is a schematic view showing mis-placement of the elements of the blood pressure measurement device according to the first embodiment of the invention;
FIGS. 5A and 5B are schematic views showing the exteriors of the blood pressure measurement device according to the preferred embodiment of the invention;
FIG. 6 is a schematic view showing a measurement process of the blood pressure measurement device according to a second preferred embodiment of the invention; and
FIG. 7 is a flow chart showing steps of the method of blood pressure measurement according to the preferred embodiment of the invention.

### DETAILED DESCRIPTION OF THE INVENTION

The present invention will be apparent from the following detailed description, which proceeds with reference to the accompanying drawings, wherein the same references relate to the same elements.

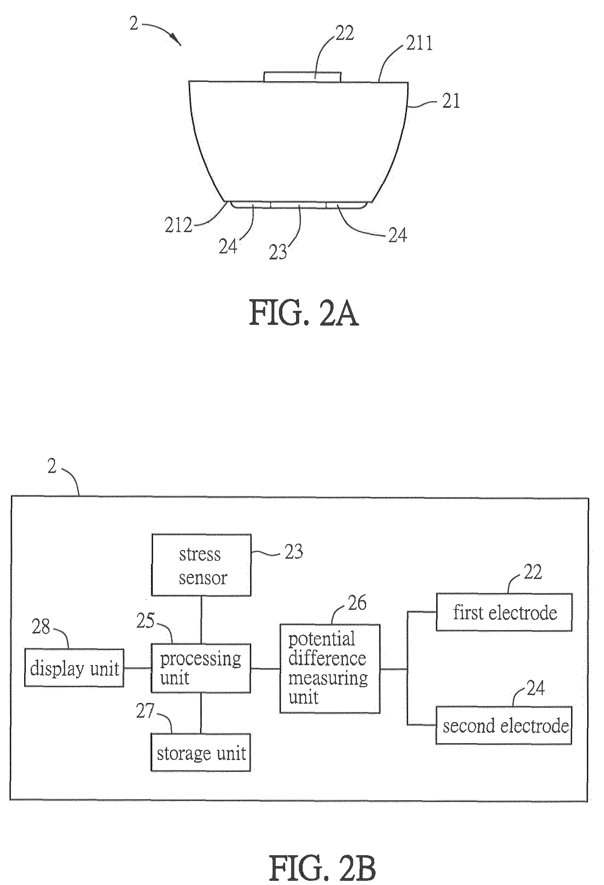

FIG. 2A is a schematic view showing an exterior of a blood pressure measurement device according to a first embodiment of the invention, and FIG. 2B is a schematic system block diagram showing the blood pressure measurement device of FIG. 2A. Referring to FIG. 2A and FIG. 2B, a blood pressure measurement device 2 comprises a case 21, a first electrode 22, a stress sensor 23 and a second electrode 24. The case 21 is for accommodating and fixing electrical elements of the blood pressure measurement device 2 and comprises a processing unit 25, a potential difference measuring unit 26, a storage unit 27 and a display unit 28. The case 21 comprises a first surface 211 and a second surface 212. When viewed from the operation angle of the user, the first surface 211 is an upper surface, and the second surface 212 is a lower surface. In this embodiment, the first surface 211 is parallel to the second surface 212. The first electrode 22 and the display unit 28 are disposed on the first surface 211, and the second electrode 24 and the stress sensor 23 are disposed on the second surface 212. The first surface 211 and the second surface 212 of the case 21 have notches so that the electrodes and the units can be firmly fixed onto the surfaces and electrically connected to the other elements inside the case 21. In addition, the notches are also provided so that each of the electrodes and the units are partially exposed outside the case 21 to achieve the object of contacting the part of the body or displaying the results.

Referring to FIG. 2B, the processing unit 25 is electrically connected to the potential difference measuring unit 26, the storage unit 27, the display unit 28 and the stress sensor 23 in this embodiment. The blood pressure measurement device 2 utilizes the processing unit 25 to access program instructions and data, required for processing, from the storage unit 27, and to perform data computation according to the program instructions to control other units to operate.

The first electrode 22 and the second electrode 24 are paired electrodes, wherein one of them is a positive electrode, and the other of them is a negative electrode, and both of them are electrically connected to the potential difference measuring unit 26. The first electrode 22 and the second electrode 24 form one set of leads, and contact different parts of the user's body, respectively. When the heart is performing the depolarization activity, the potential difference measuring unit 26 can measure one set of potential differences between the electrodes, and the processing unit 25 converts the set of potential differences into an electrocardio signal. In this embodiment, the first electrode 22 contacts the user's right limb (particularly the right-hand's index finger); and the second electrode 24 contacts the user's left limb (particularly the left-hand's wrist) to form a loop. In this embodiment, two electrodes are used to contact and measure user's left and right limbs, so the measured electrocardio signal may be referred to as a limb lead electrocardio signal. Specifically speaking, the limb lead electrocardio signal is a first limb lead (lead I) serving as a single lead electrocardio signal. In this embodiment, the electrocardio signal is an electrocardiogram (ECG) signal. However, it is to be noted that, in other embodiments of the invention, the first electrode 22 and the second electrode 24 may also contact the other parts of the body, such as the left and right legs, the part near the rib of the trunk or the part near the armpit, to measure the electrocardio signals of other leads. However, the invention is not restricted thereto.

In this embodiment, the stress sensor 23 comprises a strain gauge and a microprocessor electrically connected to the strain gauge. The strain gauge is composed of a piezoresistive material (e.g., a metal sheet) and an insulating substrate. In this embodiment, the blood pressure measurement device 2 is placed and operated on the left-hand's wrist, so the stress sensor 23 contacts the left-hand's wrist of the user and senses the pulsation of the radial artery within the wrist upon measurement of the blood pressure, so that the strain gauge deforms. The strain gauge deformation causes the variation of the resistance value, and then the microprocessor can calculate the pressure value according to the variation of the resistance value. The obtained variation of the continuous pressure values is transmitted to the processing unit 25, and then converted into the pulse signal. In other embodiments of the invention, the stress sensor 23 may also be an element made of a piezoelectric material.

While the processing unit 25 calculates the electrocardio signal through the potential difference measuring unit 26, and calculates the pulse signal through the stress sensor 23, the times of getting the two signals are also recorded, so that the pulse transmission time (PTT) can be calculated according to the time difference. In detail, the PTT is the difference between the time of appearance of the R wave (corresponding to the medium term of depolarization of the ventricular) of the electrocardio signal being judged in one heartbeat, and the time of occurrence of the pulsation of the radial artery measured on the to-be-measured part of the body (the left-hand's wrist in this embodiment). In other words, the PTT is the time duration when the pulse generated in one heartbeat travels from the heart to the left-hand's wrist. The distance from the heart to the left-hand's wrist may be a predetermined value, or may be obtained after the height of the user is manually inputted and adjusted according to a parameter. Then, the distance is divided by the PTT to obtain the pulse wave velocity (PWV), which is the velocity of the pulse, which is generated by the systole and reaches the left-hand's wrist. Thereafter, the processing unit 25 can calculate the user's blood pressures, comprising the systolic pressure and the diastolic pressure, according to the PWV through the algorithm. In this embodiment, the processing unit 25 may transmit the measured result to the display unit 28 for display. Of course, in other embodiments, the blood pressure measurement device 2 has a communication unit, which transmits the measured result to a smart phone or a tablet computer for display through bluetooth, 3G or 4G mobile communication technology or wireless communication method (e.g., Wi-Fi). However, the invention is not restricted thereto.

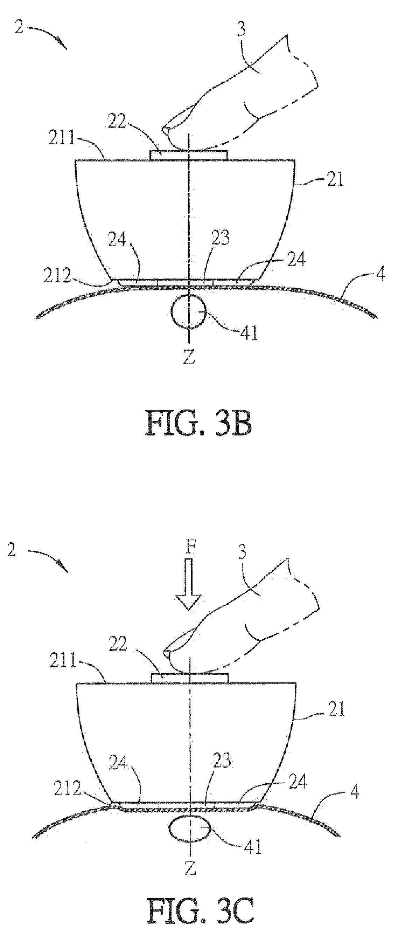

Referring to FIG. 2A, the first electrode 22 and the stress sensor 23 are disposed at upper and lower positions opposite each other in this embodiment. If viewed at the angle of the user, the first electrode 22 is disposed directly above the stress sensor 23. When the size of the first electrode 22 is slightly smaller than that of the stress sensor 23 and if the projection is made in a projection orientation Z (see FIG. 3B) perpendicular to the surface of the first electrode 22, then the projection of the first electrode 22 certainly falls within the projection of the stress sensor 23. On the contrary, if the size of the first electrode 22 is greater than the stress sensor 23 and the projection is similarly made in the projection orientation Z, then the projection of the first electrode 22 covers the projection of the stress sensor 23 on the contrary. Also, if the size of the first electrode 22 approaches the stress sensor 23 but the first electrode 22 and the stress sensor 23 have different shapes, and the projection is similarly made in the projection orientation Z, then the projections of the first electrode 22 and the stress sensor 23 may partially non-overlap but anyway may partially overlap with one another. However, the projection orientation Z is not restricted to the orientation perpendicular to the surface of the first electrode 22. In another embodiment, the projection orientation may also be the orientation perpendicular to the surface of the stress sensor 23. However, the invention is not restricted thereto.

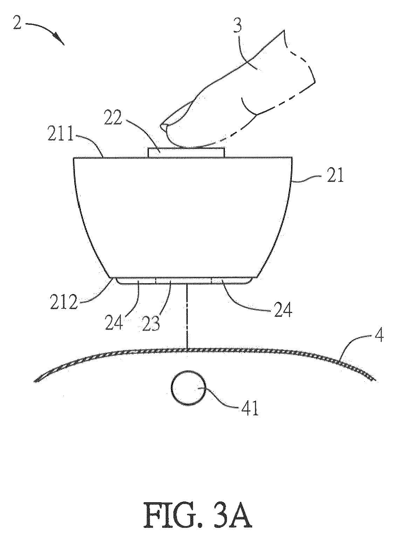

FIGS. 3A to 3C are schematic views showing operations of the blood pressure measurement device according to the first embodiment of the invention. Referring to FIG. 3A, when the user's hand holds the blood pressure measurement device 2, the user's limb 3, preferably the finger (the right-hand's index finger in this embodiment), can easily touch the first electrode 22 due to the mechanism design. Next, the user places the blood pressure measurement device 2 on the to-be-measured part 4 of the body for the preparation of measurement, as shown in FIG. 3B. Referring to FIG. 3B, the pulse signal is obtained through the measurement of the pulsation of the blood vessel 41 in the left-hand's wrist in this embodiment, so that the blood pressure can be calculated. Thus, the blood pressure measurement device 2 is placed on the left-hand's wrist. It is clear that the user's hand holds the blood pressure measurement device 2 with the gesture being kept unchanged but with the right-hand's index finger still being kept in contact with the first electrode 22.

In this embodiment, the first electrode 22 is disposed directly above the stress sensor 23, and the stress sensor 23 is further aligned with the blood vessel 41 and placed directly above the blood vessel 41. So, if the projection is made in the projection orientation Z perpendicular to the surface of the first electrode 22, then the projections of three of them at least partially overlap with one another. Furthermore, because the sizes of the stress sensor 23 and the blood vessel 41 are smaller than that of the first electrode 22 in this embodiment, the projections fall within the range of the projection of the first electrode 22.

Referring to FIG. 3C, upon the measurement of the blood pressure, the first electrode 22 is disposed directly above the stress sensor 23, the stress sensor 23 is further disposed directly above the blood vessel 41, and the first electrode 22, the stress sensor 23 and the blood vessel 41 are parallel to one another. So, when the right hand's finger of the user touches the first electrode 22, the downward pressing force generated by a gravity force exerted to the limb 3, the downward pressing force generated when the user becomes aware of the measurement and instinctively forces the finger to tightly press the first electrode 22, or the combination of the two downward pressing forces can be provided to cause an external force F. The external force F exerted on the first electrode 22 is also exerted on the stress sensor 23 with the similar magnitude, so that the stress sensor 23 is firmly and tightly pressed upon the left-hand's wrist at his/her convenience to clearly sense the pulsation of the blood vessel 41 and thus precisely obtain the pulse signal. More particularly, it is possible to overcome the problem of the measurement failure caused when the stress sensor 23 is not tightly pressed upon the wrist and the pulsation of the blood vessel 41 cannot be sensed.

In addition, the second electrode 24 of this embodiment is mounted on the periphery of the stress sensor 23. So, when the user utilizes the right hand's finger to touch the first electrode 22, he or she can make the stress sensor 23 be firmly and tightly press upon the left-hand's wrist at his/her convenience, while the second electrode 24 may also be tightly pressed upon the left-hand's wrist to ensure the formation of the loop and to ensure that the electrocardio signal can be obtained.

In the mechanism design of the blood pressure measurement device 2, because the first electrode 22, the stress sensor 23 and the blood vessel 41 at least partially overlap with one another in the projection orientation Z, an instinctive operation is caused. First, this is because that the limb 3 is kept in contact with the first electrode 22 from the time when the hand holds the blood pressure measurement device 2 to the time of starting the measurement without any movement or adjustment of the position, and that the operation is similar to the diagnosis of the pulse using the finger. Second, when viewed at the angle where the force is exerted onto the first electrode 22, the same force can be provided to the stress sensor 23 without additionally increasing the force. Third, when viewed at the angle where the force is exerted to make the stress sensor 23 be firmly pressed upon the part 4 of the body, the same force can make the limb 3 and the first electrode 22 be tightly pressed upon each other without additionally increasing the force.

In other embodiments of the invention, the user's limb 3 may be the index finger of the left or right hand, and the part 4 of the body may be any part of the body through which the blood vessel passes and the pulse can be easily obtained, wherein the part may be near the wrist, the arm, the leg, the neck or the rib of the trunk, for example. However, the invention is not restricted thereto.

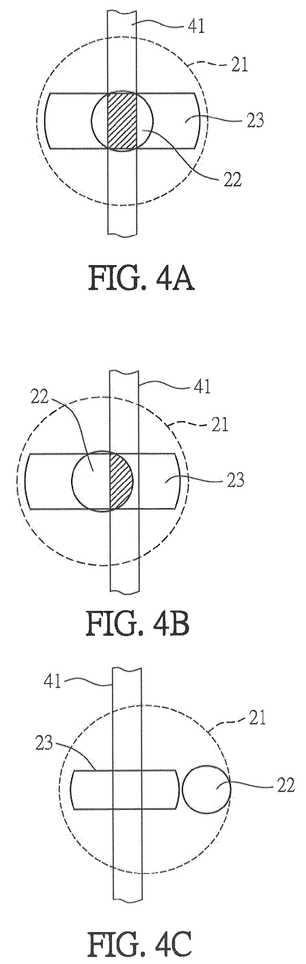

FIGS. 4A and 4B are schematic views showing elements of the blood pressure measurement device according to the first embodiment of the invention, and are provided for describing the projecting states of the first electrode 22, the stress sensor 23 and the blood vessel 41 when being projected in the projection orientation Z.

FIG. 4A shows that the first electrode 22, the stress sensor 23 and the blood vessel 41 of the blood pressure measurement device 2 according to the first embodiment of the invention completely overlap with one another in the projection orientation Z. In this embodiment, when the first electrode 22, the stress sensor 23 and the blood vessel 41 completely overlap with one another in the projection orientation Z, the user uses a limb 3 to exert an external force F to contact or press the first electrode 22, and the blood pressure measurement device 2 may also tightly and firmly press the stress sensor 23 upon the wrist through the external force F at the same time. That is, the stress sensor 23 is disposed above the blood vessel 41, so that the effect of fixing the blood pressure measurement device 2 is obtained, and it is also advantageous to the sensing of the pulsation of the blood vessel 41 and the enhancement of the accuracy of the pulse signal.

FIG. 4B shows that the first electrode 22, the stress sensor 23 and the blood vessel 41 of the blood pressure measurement device 2 according to the first embodiment of the invention partially overlap with one another in the projection orientation Z. In this embodiment, even when a little deviation of the placement position of the blood pressure measurement device 2 is present (e.g., the device 2 is not directly placed above the blood vessel of the wrist but is slightly biased leftward or rightward), the effect that the first electrode 22, the stress sensor 23 and the blood vessel 41 at least partially overlap with one another in the projection orientation Z can be maintained as long as the stress sensor 23 still faces toward the blood vessel 41. At this time, the external force F exerted from the limb 3 to the first electrode 22 still can be provided to the stress sensor 23 with the same magnitude to achieve the effect of tightly pressing the wrist at the user's convenience without deliberately increasing the force to overcome the problem of the deviation of the placement position.

On the contrary, FIG. 4C shows the assumed condition where the first electrode 22, the stress sensor 23 and the blood vessel 41 of the blood pressure measurement device 2 do not at least partially overlap with each other in the projection orientation Z. Referring to FIG. 4C, when the mechanism is not designed according to the invention (the first electrode 22 is not disposed directly above the stress sensor 23), the first electrode 22, the stress sensor 23 and the blood vessel 41 in the projection orientation Z (see FIG. 3C) do not overlap with one another. In this case, the external force F exerted from the user to the first electrode cannot assist in pressing the stress sensor 23 firmly upon the wrist, so that the pulse signal cannot be measured or the measured pulse signal is not clear. The condition of FIG. 4C becomes more obvious in the watch-type blood pressure measurement device because the stress sensor 23 and the first electrode 22 are disposed on different peripheral positions of the wrist. Referring to FIG. 4C, even if the external force F exerted on the first electrode 22 is increased, the force required to press the stress sensor 23 cannot be provided because of the components of the force. In addition, because the force has to be increased, the user may feel the inconvenience of operation, and this is not an instinctive operation.

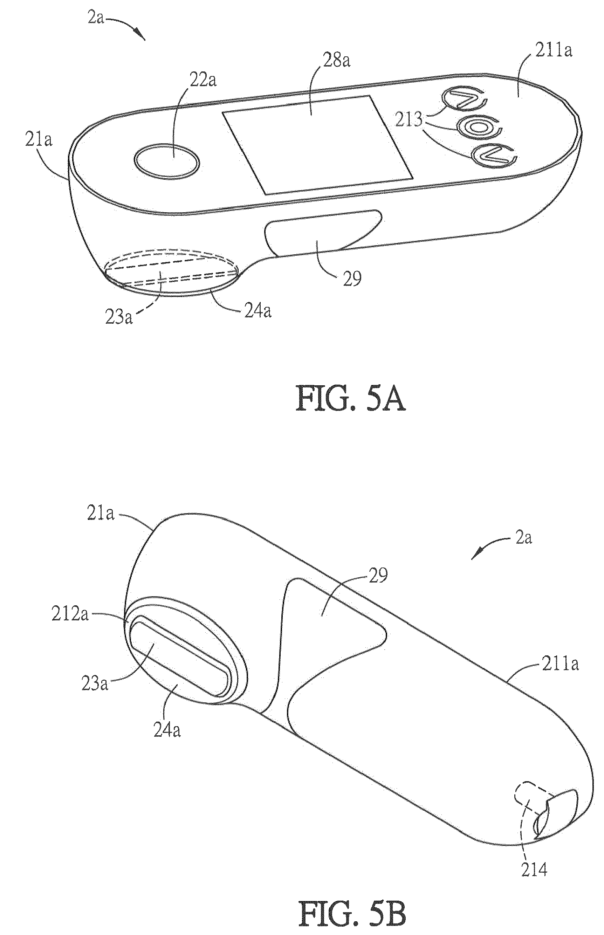

FIGS. 5A and 5B are schematic views showing the exteriors of the blood pressure measurement device according to the preferred embodiment of the invention. Referring to FIG. 5A, a blood pressure measurement device 2a of this embodiment further comprises a calibration electrode 29. The calibration electrode 29 may be disposed on a left side or a right side of a case 21a, and is electrically connected to the potential difference measuring unit in a similar manner. Upon operation, the calibration electrode 29 contacts another limb of the user, such as the thumb and the middle finger of the right hand. The potential difference measuring unit measures the potential difference between the calibration electrode 29 and the first electrode 22a at least two times within a predetermined period of time. The two measured results will be transmitted to the processing unit, and the processing unit calculates a first calibration value and a second calibration value according to the measured results. Next, the processing unit further calibrates an electrocardio signal, obtained by the measurement of the first electrode 22a and the second electrode 24a, according to an average of the first calibration value and the second calibration value. The calibration is based on the average of the calibration values to correct or remove the extreme values (e.g., the maximum and minimum value) of the electrocardio signal to obtain the electrocardio signal with the higher credibility.

Referring to FIGS. 5A and 5B, the case 21a of the blood pressure measurement device 2a of this embodiment has arced front and rear ends, and has a first surface 211 a and a second surface 212a disposed in parallel. The arced rear end of the case 21 a can make the user easily hold the blood pressure measurement device 2a, and naturally and instinctively use the limb 3a to touch the first electrode 22a and the calibration electrode 29. Thus, upon measurement of the blood pressure, the user can fix the blood pressure measurement device 2a onto the part 4a of the body in an instinctive operation manner, and then contact or even press the first electrode 22a to make the stress sensor 23a firmly and tightly press upon the part above the blood vessel.

In addition, the blood pressure measurement device 2a further comprises an input module 213, a display unit 28a and a connection port 214. The user can use the input module 213 to input the personal physiological information (comprising, for example but without limitation to, the height) to adjust the calculation parameter and enhance the measurement accuracy. The user can obtain the personal blood pressure through the display unit 28a, and may also utilize the connection port 214 to transmit the blood pressure to the electronic device to record the personal blood pressure.

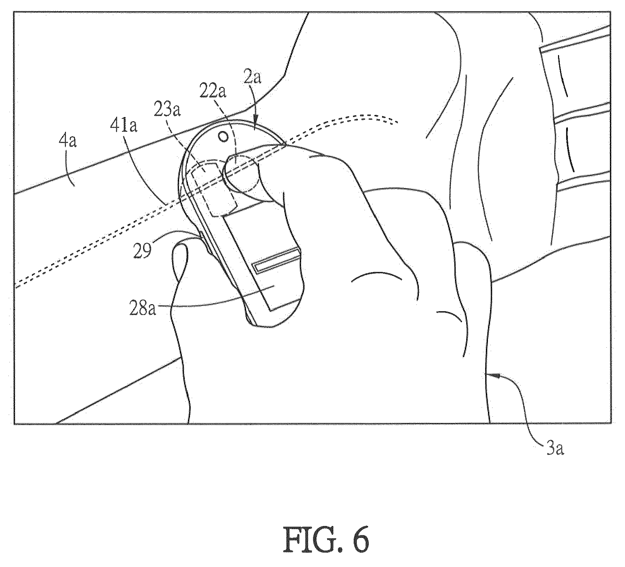

FIG. 6 is a schematic view showing an operation of a blood pressure measurement device according to a second embodiment of the invention. Upon measurement of the blood pressure, the user can easily hold the case 21a with the right-hand's index finger touching the first electrode 22a very naturally due to the mechanism design. Meanwhile, the thumb and the middle finger also naturally contact the calibration electrode 29. More importantly, the first electrode 22a, the stress sensor 23a and the blood vessel 41a overlap with one another in the projection orientation. So, when the external force exerted on the first electrode 22a by the index finger the external force exerted from the hand to the first electrode 22a due to the gravity force, or a combination of the two external forces is provided, the external force can be exerted on the stress sensor 23a with almost the same size, so that the stress sensor 23 a can be tightly pressed upon the part 4a of the body, and this is advantageous to the sensing of the pulsation of the blood vessel 41 a.

In a third embodiment of the invention, the first electrode of the blood pressure measurement device has a concave portion having a shape corresponding to the shape of the user's finger. Upon measurement of the blood pressure, the concave portion of the first electrode has the function of prompting the user to use the hand to contact or press the measurement device to operate, further enhances the effect of instinctively operating the blood pressure measurement device, and further assists in fixing the position of the finger so that the user can exert the force more easily.

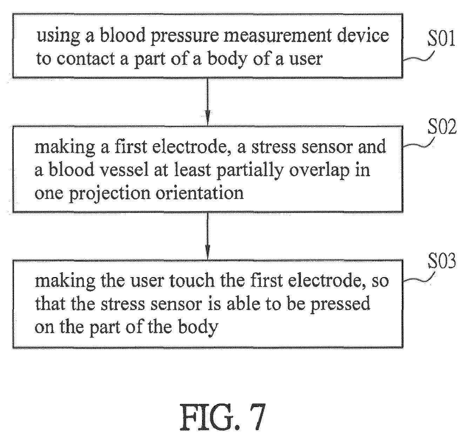

FIG. 7 is a flow chart showing steps of a method of blood pressure measurement according to the preferred embodiment of the invention. Referring to FIG. 7, the method of blood pressure measurement according to this embodiment comprises the following steps. First, a blood pressure measurement device contacts a part of a body of a user (step SO1). The blood pressure measurement device comprises a case, a first electrode and a stress sensor. The case has a first surface and a second surface. The first electrode is disposed on the first surface. The stress sensor is disposed on the second surface, and the part of the body has a blood vessel inside, so that the first electrode, the stress sensor and the blood vessel at least partially overlap in one projection orientation (step S02). The user touches the first electrode to make the stress sensor be able to be pressed on the part of the body (step S03). However, the contents and implementation details of the method of blood pressure measurement of this embodiment are almost the same as those of the technology executed by the measurement device, and can be easily found hereinabove. So, detailed descriptions thereof will be omitted.

In summary, the invention provides a blood pressure measurement device and a method of blood pressure measurement, in which the blood pressure is calculated according to the two electrodes and the sensing signal of the stress sensor without the need of the host and the cuff for pressurization and depressurization. Thus, the size can be advantageously decreased so that the blood pressure measurement device can be easily carried, and the user's desire of monitoring the blood pressure can be enhanced. More importantly, the mechanism is designed such that the first electrode, the stress sensor and the blood vessel in the to-be-measured part of the body at least partially overlap in one projection orientation. Thus, when the user is operating the device, the user only needs to touch the first electrode at his/her convenience to make the stress sensor press upon the to-be-measured part of the body. Thus, the measurement precision can be enhanced, and the requirement of the subsequent calibration using the software is decreased. More particularly, an instinctive operation can be provided to achieve the good measurement effect without the particular learning.

Although the invention has been described with reference to specific embodiments, this description is not meant to be construed in a limiting sense. Various modifications of the disclosed embodiments, as well as alternative embodiments, will be apparent to persons skilled in the art. It is, therefore, contemplated that the appended claims will cover all modifications that fall within the true scope of the invention.

## Claims

1. A blood pressure measurement device, comprising:
a case comprising a first surface and a second surface;
a first electrode disposed on the first surface; and
a stress sensor disposed on the second surface,
wherein the blood pressure measurement device is operated on a part of a body of a user, the part of the body has a blood vessel inside, and upon measurement of a blood pressure, the first electrode, the stress sensor and the blood vessel at least partially overlap in one projection orientation, so that when the user touches the first electrode, the stress sensor is able to be pressed on the part of the body.

2. The blood pressure measurement device according to claim 1, wherein when the user touches the first electrode, the stress sensor encounters a downward pressing force generated by a gravity force exerted to the user or an exertion force exerted by the user and is able to be pressed on the part of the body.

3. The blood pressure measurement device according to claim 1, further comprising:
a second electrode, which is disposed on the second surface and is electrically connected to the first electrode, and upon measurement of the blood pressure, the second electrode contacts the part of the body.

4. The blood pressure measurement device according to claim 3, wherein when the user touches the first electrode, the second electrode is able to be pressed on the part of the body.

5. The blood pressure measurement device according to claim 3, further comprising:
a calibration electrode disposed on the case;
one or multiple processing units accommodated within the case; and
a storage unit, which is accommodated within the case and is signal connected to the one or multiple processing units, wherein the storage unit comprises one or multiple program instructions, and when the one or multiple program instructions are executed by the one or multiple processing units, the one or multiple processing units execute the steps of:
obtaining a first calibration value, wherein the first calibration value is calculated according to a first measured result of the calibration electrode and of the first electrode;
obtaining a second calibration value, wherein the second calibration value is calculated according to a second measured result of the calibration electrode and the first electrode; and
calibrating measured values obtained by the first electrode and the second electrode according to the first calibration value and the second calibration value.

6. The blood pressure measurement device according to claim 1, wherein the user uses a finger to touch the first electrode, and the first electrode has a concave portion corresponding to a shape of the finger.

7. A method of blood pressure measurement, the method comprising the steps of:
using a blood pressure measurement device to contact a part of a body of a user, wherein the blood pressure measurement device comprises a case, a first electrode and a stress sensor, the case comprises a first surface and a second surface, the first electrode is disposed on the first surface, the stress sensor is disposed on the second surface, and the part of the body has a blood vessel inside;
making the first electrode, the stress sensor and the blood vessel at least partially overlap in one projection orientation; and
making the user touch the first electrode so that the stress sensor is able to be pressed on the part of the body.

8. The method of blood pressure measurement according to claim 7, wherein when the user touches the first electrode, the stress sensor encounters a downward pressing force generated by a gravity force exerted to the user or an exertion force exerted by the user and is able to be pressed on the part of the body.

9. The method of blood pressure measurement according to claim 7, wherein the blood pressure measurement device further comprises a second electrode disposed on the second surface and electrically connected to the first electrode, and upon measurement of the blood pressure, the second electrode contacts the part of the body.

10. The method of blood pressure measurement according to claim 9, wherein when the user touches the first electrode, the second electrode is able to be pressed on the part of the body.

11. The method of blood pressure measurement according to claim 9, wherein the blood pressure measurement device further comprises a calibration electrode disposed on the case, one or multiple processing units accommodated within the case, and a storage unit accommodated within the case and signal connected to the one or multiple processing units, the storage unit comprises one or multiple program instructions, and when the one or multiple program instructions are executed by the one or multiple processing units, the one or multiple processing units execute a calibration procedure comprising steps of:
obtaining a first calibration value, wherein the first calibration value is calculated according to a first measured result of the calibration electrode and the first electrode;
obtaining a second calibration value, wherein the second calibration value is calculated according to a second measured result of the calibration electrode and the first electrode; and
calibrating measured values obtained by the first electrode and the second electrode according to the first calibration value and the second calibration value.

12. The method of blood pressure measurement according to claim 7, wherein the user uses a finger to touch the first electrode, and the first electrode has a concave portion corresponding to a shape of the finger.

## Amended claims

### Amended claims in accordance with Rule 137(2) EPC.

1. A blood pressure measurement device (2, 2a), comprising:
a case (21, 21a) comprising a first surface (211, 211 a) and a second surface (212, 212a);
a first electrode (22, 22a) disposed on the first surface (211, 211a); and
a stress sensor (23, 32a) disposed on the second surface (212, 212a),
wherein the blood pressure measurement device (2, 2a) is configured to be operated on a part of a body of a user, the part of the body has a blood vessel inside, and upon measurement of a blood pressure, the first electrode (22, 22a), the stress sensor (23, 23a) and the blood vessel at least partially overlap in one projection orientation, so that when the user touches the first electrode (22, 22a), the stress sensor (23, 23a) is able to be pressed on the part of the body.

2. The blood pressure measurement device (2, 2a) according to claim 1, wherein when the user touches the first electrode (22, 22a), the stress sensor (23, 23a) encounters a downward pressing force generated by a gravity force exerted to the user or an exertion force exerted by the user and is able to be pressed on the part of the body.

3. The blood pressure measurement device (2, 2a) according to claim 1, further comprising:
a second electrode (24, 24a), which is disposed on the second surface (212, 212a) and is electrically connected to the first electrode (22, 22a), and upon measurement of the blood pressure, the second electrode (24, 24a) contacts the part of the body.

4. The blood pressure measurement device (2, 2a) according to claim 3, wherein when the user touches the first electrode (22, 22a), the second electrode (24, 24a) is able to be pressed on the part of the body.

5. The blood pressure measurement device (2a) according to claim 3, further comprising:
a calibration electrode (29) disposed on the case (21a);
one or multiple processing units (25) accommodated within the case (21a); and
a storage unit (27), which is accommodated within the case (21a) and is signal connected to the one or multiple processing units (25), wherein the storage unit (27) comprises one or multiple program instructions, and when the one or multiple program instructions are executed by the one or multiple processing units (25), the one or multiple processing units (25) execute the steps of:
obtaining a first calibration value, wherein the first calibration value is calculated according to a first measured result of the calibration electrode (29) and of the first electrode (22a);
obtaining a second calibration value, wherein the second calibration value is calculated according to a second measured result of the calibration electrode (29) and the first electrode (22a); and
calibrating measured values obtained by the first electrode (22a) and the second electrode (24a) according to the first calibration value and the second calibration value.

6. The blood pressure measurement device (2, 2a) according to claim 1, wherein the user uses a finger to touch the first electrode (22, 22a), and the first electrode (22, 22a) has a concave portion corresponding to a shape of the finger.

7. A method of blood pressure measurement, the method comprising the steps of:
using (S01) a blood pressure measurement device (2, 2a) to contact a part of a body of a user, wherein the blood pressure measurement device (2, 2a) comprises a case (21, 21a), a first electrode (22, 22a) and a stress sensor (23, 23a), the case (21, 21a) comprises a first surface (211, 211a) and a second surface (212, 212a), the first electrode (22, 22a) is disposed on the first surface (211, 211a), the stress sensor (23, 23a) is disposed on the second surface (212, 212a), and the part of the body has a blood vessel inside;
making (S02) the first electrode (22, 22a), the stress sensor (23, 23a) and the blood vessel at least partially overlap in one projection orientation; and
making (S03) the user touch the first electrode (22, 22a) so that the stress sensor (23, 23a) is able to be pressed on the part of the body.

8. The method of blood pressure measurement according to claim 7, wherein when the user touches the first electrode (22, 22a), the stress sensor (23, 23a) encounters a downward pressing force generated by a gravity force exerted to the user or an exertion force exerted by the user and is able to be pressed on the part of the body.

9. The method of blood pressure measurement according to claim 7, wherein the blood pressure measurement device (2, 2a) further comprises a second electrode (24, 24a) disposed on the second surface (212, 212a) and electrically connected to the first electrode (22, 22a), and upon measurement of the blood pressure, the second electrode (24, 24a) contacts the part of the body.

10. The method of blood pressure measurement according to claim 9, wherein when the user touches the first electrode (22, 22a), the second electrode (24, 24a) is able to be pressed on the part of the body.

11. The method of blood pressure measurement according to claim 9, wherein the blood pressure measurement device (2a) further comprises a calibration electrode (29) disposed on the case (21a), one or multiple processing units (25) accommodated within the case (21 a), and a storage unit (27) accommodated within the case (21a) and signal connected to the one or multiple processing units (25), the storage unit (27) comprises one or multiple program instructions, and when the one or multiple program instructions are executed by the one or multiple processing units (25), the one or multiple processing units (25) execute a calibration procedure comprising steps of:
obtaining a first calibration value, wherein the first calibration value is calculated according to a first measured result of the calibration electrode (29) and the first electrode (22a);
obtaining a second calibration value, wherein the second calibration value is calculated according to a second measured result of the calibration electrode (29) and the first electrode (22a); and
calibrating measured values obtained by the first electrode (22a) and the second electrode (24a) according to the first calibration value and the second calibration value.

12. The method of blood pressure measurement according to claim 7, wherein the user uses a finger to touch the first electrode (22a), and the first electrode (22a) has a concave portion corresponding to a shape of the finger.
